# EUROPÄISCHE PATENTANMELDUNG

(11) **EP 1 362 635 A1**
(43) Veröffentlichungstag der Anmeldung: **19.11.2003**
(21) Anmeldenummer: 02010806.4
(22) Anmeldetag: 15.05.2002
(51) Int. Cl.: B01F 7/00, B01F 7/24, B01F 3/04, C02F 3/12, C02F 11/04, C12M 1/02, C12M 1/107, C02F 3/30

(54) **Rühreinrichtung für einen Fermenter einer Biogasanlage**

(71) Anmelder: U.T.S. Umwelt-Technik-Süd GmbH, 84419 Obertaufkirchen (DE)
(72) Erfinder: Bürger, Adam, 84405 Grüntegernbach (DE)
(74) Vertreter: Liebl, Thomas, Dipl.-Ing.

(57) **Zusammenfassung**

Die Erfindung betrifft eine Rühreinrichtung (1) für einen Fermenter einer Biogasanlage, die in einem Fermenterbehälterinnenraum (2) eines Fermenterbehälters (3) zum Umwälzen einer mit Biomasse-Feststoffen versetzten Fermenterflüssigkeit (12) anordenbar ist. Erfindungsgemäß ist die Rühreinrichtung (1) durch eine in einem Schneckengehäuse (7) aufgenommene und mittels einem Schneckenantrieb (8) in eine Vorwärtsrichtung und/oder eine Rückwärtsrichtung antreibbare Schnecke (9) gebildet. Am Schneckengehäuse (7) ist eine erste Gehäuseöffnung (10; 28) und eine davon in Gehäuselängsrichtung beabstandete, zweite Gehäuseöffnung (11) vorgesehen, wobei wenigstens eine der beiden Gehäuseöffnungen (10, 11; 28) im montierten Zustand der Rühreinrichtung (1) in die mit Biomasse-Feststoffen versetzte Fermenterflüssigkeit (12) eintaucht.

## Beschreibung

Die Erfindung betrifft eine Rühreinrichtung für einen Fermenter einer Biogasanlage nach dem Oberbegriff des Anspruchs 1.

In Biogasanlagen läuft ein Fermentationsprozess ab, bei dem organische Stoffe, wie beispielsweise Wirtschaftsdünger aus der Landwirtschaft (Rindergülle, Rinder-Festmist, Schweinegülle, Schweine-Festmist, Hühnergülle, Hühner-Trockenkot) und/oder landwirtschaftliche Reststoffe (Grasschnitt-Rübenblätter, Silagen) und/oder Reststoffe aus der Agroindustrie oder verwandten Industrien (Biertreber, Obstreste, Gemüsereste, Rapsschrot, Getreideabputz, Schlempen, Melasse) als Biomasse vergast werden. Die hierbei entstehenden Gase sammeln sich in einem oberen Fermenterbehälterbereich eines Fermenterbehälters und können direkt zur Energieerzeugung verwendet werden, z. B. als Heizgas zur Stromerzeugung in nachgeschalteten Brennkraftmaschinen mit Elektrogeneratoren. Zur Fermentation werden im Fermenterbehälter die organischen Stoffe mit Flüssigkeit versetzt und Mikroorganismen, wie z. B. Hefen, Bakterien etc. zugeführt, so dass der Fermentations- bzw. der Vergasungsprozess unter aeroben oder anaeroben Bedingungen ablaufen kann.

Ein Problem bei derartigen allgemein bekannten Fermentationsprozessen ist, dass die Biomasse-Feststoffe in der Fermenterflüssigkeit regelmäßig nicht gleichmäßig verteilt sind, da sie z. B. aufschwimmen und sich im Bereich der Flüssigkeitsoberfläche ansammeln, was z. B. der Fall ist, wenn das spezifische Gewicht der Biomasse-Feststoffe geringer ist als dasjenige der Flüssigkeit, z. B. Wasser. Andererseits besteht aber auch das Problem, dass Biomasse-Feststoffe mit einem höheren spezifischen Gewicht als die Flüssigkeit auf den Fermenterbehälterboden absinken und sich dort als Sinkschichten ansammeln. Derartige Ansammlungen von Biomasse-Feststoffen bewirken eine ungleichmäßige Biomasseverteilung in der Fermenterflüssigkeit, was sich wiederum nachteilig auf den Wirkungsgrad des Fermentationsprozesses auswirkt.

Für eine gleichmäßigere Verteilung der Biomasse-Feststoffe sind bereits Tauchmotorrührgeräte bekannt, die mittels einer Seilwinde entlang eines Aggregatträgers verfahrbar sind. So ist aus der gattungsgemäßen DE 197 32 198 C1 eine Rühreinrichtung für einen Fermenter einer Biogasanlage bekannt, die in einem Fermenterbehälterinnenraum eines Fermenterbehälters zum Umwälzen einer mit Biomasse-Feststoffen versetzten Fermenterflüssigkeit anordenbar ist.

Konkret ist die Rühreinrichtung hier durch ein Tauchmotorrührgerät gebildet, das an einem vertikal im Behälter angeordneten Aggregatträger höhenverstellbar gehalten ist. Eine Seiltrommel ist hier innerhalb im oberen Bereich des Fermenterbehälters angeordnet, so dass keine gasdichte Seildurchführung durch eine Fermenterbehälterwand erforderlich ist. Eine außerhalb des Behälters angeordnete Betätigungseinrichtung für den Seiltrommelantrieb ist mit dem mittels einer dichten Durchführung durch eine Behälterwand geführten Seiltrommelantrieb verbunden. Das Tauchmotorrührgerät wird hier durch einen Tauchmotor gebildet, der einen herkömmlichen Propellerrührer antreibt. Bei dem Tauchmotor handelt es sich um einen Elektromotor, der mit elektrischen Leitungen verbunden ist, die von außerhalb des Behälters in den Fermenterbehälterinnenraum geführt werden müssen. Dies ist in gewisser Weise problematisch, da sich im Fermenterbehälterinnenraum aggressive Gas- und/oder Flüssigkeitsmedien befinden, die die elektrischen Leitungen angreifen und dadurch gegebenenfalls beschädigen können. Aus Explosionsschutzgründen ist zudem die Anwesenheit von elektrischen Leitungen im eine Gasphase aufweisenden Fermenterbehälterinnenraum problematisch. Dies erfordert daher aus sicherheitstechnischen Gründen eine dauerhafte, zeitaufwendige Kontrolle der Anlage. Zudem bewirkt eine derartige Rühreinrichtung mit einem Propellerrührer nur eine Vermischung in einem begrenzten Bereich innerhalb der Fermenterflüssigkeit, so dass es für eine bessere Durchmischung erforderlich ist, das Tauchmotorrührgerät mittels dem Aggregatträger um dessen Längsachse zu verschwenken, damit der Propellerrührer auch andere bezüglich der Feststoffansammlungen kritische Fermenterflüssigkeitsbereiche durchmischt. Hierzu sind insbesondere im Hinblick auf die Seiltrommelanordnung komplizierte Getriebemechaniken und/oder Gelenkanordnungen erforderlich, um das Verschwenken des Tauchmotorrührgerätes um den Aggregatträger zu ermöglichen. Ein weiteres Problem in Verbindung mit einer derartigen Rühreinrichtung ist, dass sich z. B. langfasrige Biomasse-Feststoffe beim Betrieb des Propellerrührers so um diesen herumwickeln können, dass dessen Betätigung blockiert wird. Damit ist die Funktionssicherheit der Rühreinrichtung insgesamt gefährdet.

Aufgabe der Erfindung ist es daher, eine Rühreinrichtung für einen Fermenter einer Biogasanlage zu schaffen, die bei einfachem Aufbau eine sehr gute Durchmischung der Biomasse-Feststoffe in der Fermenterflüssigkeit bewirkt, die vielfältigst einsetzbar ist, und die eine hohe Funktionssicherheit aufweist.

Diese Aufgabe wird gelöst mit den Merkmalen des Anspruchs 1.

Gemäß Anspruch 1 ist die Rühreinrichtung durch eine in einem Schneckengehäuse aufgenommene und mittels einem Schneckenantrieb in eine Vorwärtsrichtung und/oder eine Rückwärtsrichtung antreibbare Schnecke gebildet. Am Schneckengehäuse ist eine erste Gehäuseöffnung und eine davon in Gehäuselängsrichtung beabstandete, zweite Gehäuseöffnung vorgesehen, wobei wenigstens eine der beiden Gehäuseöffnungen im montierten Zustand der Rühreinrichtung in die mit Biomasse-Feststoffen versetzte Fermenterflüssigkeit eintaucht.

Mit einem derartigen Aufbau der Rühreinrichtung durch eine in einem Schneckengehäuse aufgenommene Schnecke wird eine Rühreinrichtung geschaffen, die bei einfachem Aufbau eine sehr gute und gleichmäßige sowie auf die jeweils gegebenen Probleme (Schwimmschichten, Sinkschichten) optimal einstellbare Verteilung der Biomasse-Feststoffe in der Fermenterflüssigkeit durch Umwälzung ermöglicht. Dies wird nachfolgend insbesondere anhand der Merkmale der Ansprüche 2 und 3 deutlich:
So kann gemäß Anspruch 2 in einer ersten Betriebsart die Rühreinrichtung so im Fermenterbehälter montiert sein, dass die zweite Gehäuseöffnung als untere Gehäuseöffnung in die Fermenterflüssigkeit eingetaucht ist, während die erste Gehäuseöffnung als obere Gehäuseöffnung im Gasbereich oberhalb des Flüssigkeitsspiegels, vorzugsweise unmittelbar oberhalb des Flüssigkeitsspiegels, angeordnet ist. Dadurch strömt bei einem Antrieb der Schnecke mittels dem Schneckenantrieb in die Rückwärtsrichtung über die untere Gehäuseöffnung ein Fermenterflüssigkeitsanteil mitsamt Biomasse-Feststoffen im Kreislauf in das Schneckengehäuse ein und wird dann mittels der Schnecke in den Bereich der oberen Gehäuseöffnung gefördert, über die der mit Biomasse-Feststoffen versetzte Fermenterflüssigkeitsanteil wieder aus dem Schneckengehäuse ausströmt, um diesen wieder der Fermenterflüssigkeit im Fermenterbehälterinnenraum zuzuführen. Dadurch wird somit im Fermenterbehälterinnenraum eine bezogen auf das Schneckengehäuse Vertikalströmung als Tumbleströmung in der Fermenterflüssigkeit ausgebildet, die entgegen den Uhrzeigersinn gerichtet ist. Mit einer derartigen Vertikalströmung kann eine besonders vorteilhafte gleichmäßige Verteilung der Biomasse-Feststoffe in der Fermenterbehälterflüssigkeit erreicht werden, was sich wiederum positiv auf den Wirkungsgrad der gesamten Fermentation auswirkt. Und dies zum einen durch die Vertikalströmung in der Fermenterflüssigkeit und zum anderen auch durch die Förderung mittels der Schnecke im Schneckengehäuse selbst. Zudem ist durch die Schnecke sichergestellt, dass die Rühreinrichtung nicht durch z. B. langfasrige Feststoffe blockiert werden kann, wobei die Schnecke vorzugsweise die Biomasse-Feststoffe regelmäßig auch noch zerkleinert. Dadurch ist auch die Funktionssicherheit des Betriebs der Rühreinrichtung insgesamt gegenüber den herkömmlichen Lösungen mittels einem Propellerrührer und Tauchmotor wesentlich erhöht.

Gemäß einer weiteren Betriebsart nach Anspruch 3 tauchen beide Gehäuseöffnungen im montierten Zustand der Rühreinrichtung in die Fermenterflüssigkeit ein. Dadurch wird bei einem Antrieb der Schnecke in die Vorwärtsrichtung erreicht, dass über die obere Gehäuseöffnung ein mit Biomasse-Feststoffen versetzter Fermenterflüssigkeitsanteil im Kreislauf in das Schneckengehäuse einströmt und mittels der Schnecke in den Bereich der unteren Gehäuseöffnung förderbar ist. Über diese untere Gehäuseöffnung strömt dann der mit Biomasse-Feststoffen versetzte Fermenterflüssigkeitsanteil wieder aus dem Schneckengehäuse heraus in die Fermenterflüssigkeit im Fermenterbehälterinnenraum. Hierdurch wird somit wiederum eine bezogen auf das Schneckengehäuse Vertikalströmung als Tumbleströmung in der Fermenterflüssigkeit ausgebildet, die entgegen der vorher geschilderten Betriebsweise nunmehr in Uhrzeigersinn verläuft. Alternativ zu dieser Betriebsweise kann jedoch durch einen Antrieb der Schnecke in Rückwärtsrichtung dann hier auch wahlweise eine Vertikalströmung entgegen den Uhrzeigersinn erzeugt werden.

Mit der erfindungsgemäßen Rühreinrichtung lässt sich somit eine auf die jeweiligen Gegebenheiten angepasste Vertikalströmung in der Fermenterflüssigkeit zur gleichmäßigen Verteilung der Biomasse-Feststoffe in der Fermenterflüssigkeit erzeugen. So lassen sich mit der Tumbleströmung im Uhrzeigersinn, bei der über die obere Gehäuseöffnung ein mit Biomasse-Feststoffen versetzter Flüssigkeitsanteil angesaugt wird, vorteilhaft obere Schwimmschichten im Bereich der Flüssigkeitsoberfläche in den unteren Flüssigkeitsbereich umverteilen. Andererseits wird bei einem Rückwärtsantrieb der Schnecke und dem dadurch bedingten Ansaugen des mit Biomasse-Feststoffen versetzten Fermenterflüssigkeitsanteils über die untere Gehäuseöffnung erreicht, dass Sinkschichten nach oben in den oberen Flüssigkeitsbereich gebracht werden können. Wie bereits erwähnt, kann dabei zusätzlich zur Ausbildung einer derartigen vorteilhaften Vertikalströmung durch die Schnecke auch eine vorteilhafte Zerkleinerung der Biomasse-Feststoffe erfolgen, was sich wiederum vorteilhaft auf den Fermentationsprozess insgesamt auswirkt. Dazu ist die Schnecke vorzugsweise wenigstens teilweise aus einem harten Material ausgebildet, z. B. an den Außenkanten der Wendel beziehungsweise an den Schraubflächen der Schnecke.

Grundsätzlich kann der Schneckenantrieb, z.B. ein Elektromotor mit variabler Drehzahlsteuerung, außerhalb des Fermenterbehälters angeordnet werden, wozu lediglich ein Antriebsstrang der Schnecke dicht durch eine Behälterwand geführt werden muss, z. B. durch eine obere Behälterwand als Behälterdecke, was einfach zu bewerkstelligen ist. Besonders bevorzugt ist jedoch auch ein Aufbau, bei der der Schneckenantrieb im Fermenterbehälter angeordnet und mittels einem Arbeitsmedium antreibbar ist. Dieser mittels einem Arbeitsmedium antreibbare Schneckenantrieb ist mit einer Energieleitung verbunden, die einerseits eine Rohr- und/oder Schlauchleitung als Zuführleitung für das Medium aufweist, die an eine Medium-Kompressionseinheit angeschlossen ist und die dicht durch eine Behälterwand zum Schneckenantrieb geführt ist. Weiter umfasst die Energieleitung eine Rohr- und/oder Schlauchleitung als Abführleitung für das Medium, die dicht durch eine Behälterwand aus dem Fermenterbehälter herausgeführt ist. Die Medium-Kompressionseinheit ist elektrisch betreibbar und außerhalb des Fermenterbehälters angeordnet. Gemäß einer ersten konkreten Ausführungsform hierzu kann der Schneckenantrieb ein Hydraulik-Motor sein, wobei das Medium eine Hydraulikflüssigkeit ist und die Kompressionseinheit ein Hydraulik-Kompressor ist. Die Abführleitung ist dabei als Rückleitung in einem geschlossenen Kreislauf an den Hydraulik-Kompressor angeschlossen. Alternativ dazu kann der Schneckenantrieb auch durch einen Pneumatik-Motor gebildet sein, wobei als Arbeitsmedium hier dann Luft verwendet ist und die Kompressionseinheit als Luftkompressor ausgebildet ist. Bei dieser Anordnung reicht es aus, wenn die Abführleitung als Abblasleitung lediglich dicht aus dem Fermenterbehälter geführt ist. Bei Verwendung eines anderen Gases, beispielsweise eines Inertgases, kann auch hier ein geschlossener Gaskreislauf mit einer Gasrückführung zum Luftkompressor eingesetzt werden. Sowohl in der Hydraulik- als auch in der Pneumatikausführung ergeben sich die Vorteile, dass die elektrisch betriebenen Kompressoren außerhalb des Fermenterbehälters angeordnet sind und somit der insbesondere aufgrund der Gasphase erforderliche Explosionsschutz durch die Verwendung von Hydraulik- oder Gasleitungen sowie die entsprechenden Hydraulik- oder Gasmotoren einfach beherrschbar ist. Gegenüber einer elektrischen Energieversorgung mit Elektrokabeln ist zumindest im Fermenterbehälter ein Zwei-Leitungssystem mit Rohr- und/oder Schlauchleitungen als Medium-Zuführleitung und Medium-Abführleitung erforderlich. Insbesondere bei Verwendung eines Pneumatik-Motors ist ein sonst übliches Abblasen von Luft unmittelbar am Motor nicht möglich, da diese Luft in den Flüssigkeitsbereich und dann in den Bereich der Gasphase gelangen würde und dort zu einer unerwünschten Verdünnung des Biogases und einer Gasvermehrung führen würde.

Die Vertikalströmung bildet sich dabei je nach Dimensionierung des Fermenterbehälters und je nach Dimensionierung der Rühreinrichtung im Wesentlichen im gesamten Fermenterflüssigkeitsbereich aus, so dass im Wesentlichen der gesamten Fermenterflüssigkeitsbereich umwälzbar ist. Dies wird insbesondere dadurch erreicht, dass die Ausströmgeschwindigkeit des mit Biomasse-Feststoffen versetzten Flüssigkeitsanteils im Bereich der Gehäuseöffnungen so groß ist, dass dieser bis an die Behälterwand gespült wird und dort nach oben steigt beziehungsweise unten sinkt und im Kreislauf wieder über die jeweils andere Gehäuseöffnung eingesaugt wird. Dies wird zudem auch dadurch erreicht, dass die beiden für die Ausbildung einer Vertikalströmung relevanten Gehäuseöffnungen möglichst weit voneinander beabstandet sind, z. B. bei einer vertikalen Anordnung der Schnecke einmal im Bereich der Flüssigkeitsoberfläche und andererseits im gegenüberliegenden Bodenbereich ausgebildet sind. Eine derartige als Tumbleströmung bezeichnete Vertikalströmung bewirkt somit eine besonders effektive Durchmischung der mit vielen Biomasse-Feststoffen versetzten Fermenterflüssigkeit. Zudem bildet sich eine derartige Vertikalströmung nicht nur in eine Richtung aus, sondern bei einem frei angeordneten Schneckengehäuse in Umfangsrichtung um das gesamte Schneckengehäuse herum. Vorliegend wurden die Vorteile der Rührereinrichtung im bevorzugten Betrieb mit hohem Feststoffanteil in der Fermenterflüssigkeit geschildert. Selbstverständlich ist eine derartige erfindungsgemäße Rühreinrichtung jedoch auch zur Vermischung von Flüssigkeiten geeignet, in denen kaum oder nur wenig Feststoffanteil vorhanden ist.

Bevorzugt ist dabei die Schnecke mitsamt Schneckengehäuse im montierten Zustand in etwa vertikal ausgerichtet im Fermenterbehälterinnenraum angeordnet. Dadurch kann z. B. der Antriebsstrang nach außen durch die Fermenterbehälterdecke geführt werden, so dass dieser einfach zugänglich ist. Zudem ist eine derartige Anordnung insbesondere auch im Hinblick auf eine eventuell vorhandene Höhenverstellung der Rühreinrichtung vorteilhaft, da diese dann einfachst als Vertikalverstellung erfolgt.

Das Schneckengehäuse weist vorzugsweise einen Rohrquerschnitt auf. Ein derartiges Schneckengehäuse ist einfach und preiswert herstellbar und besonders gut geeignet zur formschlüssigen Aufnahme einer Schnecke. Das Schneckengehäuse kann z.B. einen Kreisrohrquerschnitt aufweisen. Besonders vorteilhaft ist jedoch ein Schneckengehäuse mit einem eckigen oder ovalen Rohrquerschnitt, da damit in der Flüssigkeit vorhandene Fremdkörper, z.B. größere stabile Teile, durch den unterschiedlichen Abstand der Schnecke zum Schneckengehäuse ohne Verklemmen und damit ggfs. Blockieren der Schnecke besser ausweichen können. Alternativ oder zusätzlich dazu können an den Schneckenaußenkanten hierfür auch Ausnehmungen vorgesehen sein, z.B. halbmondförmige Ausnehmungen. Das Schneckengehäuse ist ebenso wie die Schnecke vorzugsweise aus einem stabilen Material, z.B. einem gegen aggressive Medien widerstandsfähigen Metallwerkstoff ausgebildet.

Die beiden Gehäuseöffnungen können grundsätzlich durch eine endseitige Rohröffnung des Schneckengehäuses ausgebildet sein oder alternativ dazu aber auch in einem Seitenwandbereich des Schneckengehäuses ausgebildet sein. Auch eine Ausbildung einer Gehäuseöffnung im Seitenwandbereich und einer weiteren Gehäuseöffnung durch eine endseitige Rohröffnung ist jederzeit möglich. Ersichtlich ist dadurch ein vielseitiger Aufbau für einen universellen Einsatzbereich der erfindungsgemäßen Rühreinrichtung möglich.

Besonders gute Ergebnisse insbesondere im Hinblick auf die Verteilung der Biomasse-Feststoffe in der Fermenterflüssigkeit ergeben sich dadurch, dass im Bereich der in die Fermenterflüssigkeit eintauchenden Gehäuseöffnung ein Auslaufstutzen angeordnet ist, der z. B. bei einer vertikalen Schneckenausrichtung um 90° gegenüber der Schnecke abgewinkelt ist. Dieser Auslaufstutzen kann integral mit dem Schneckengehäuse ausgebildet sein oder aber auch als separates Bauteil auf dieses aufsetzbar sein, um gegebenenfalls unterschiedliche Auslaufstutzen aufzusetzen. Beispielsweise kann dadurch der Vertikalströmung eine bestimmte Strömungsrichtung vorgegeben werden, so dass eine noch bessere individuelle Anpassung an die jeweils konkret gegebenen Erfordernisse im Hinblick auf die Vermischung der Fermenterflüssigkeit möglich ist.

Beispielsweise können auch mehrere derartige Auslaufstutzen in einer Auslaufstutzen-Horizontalebene liegend vorgesehen sein, die voneinander mit vorgebbaren Winkelabständen beabstandet sind. Mit einer derartigen Anordnung lässt sich zudem gegebenenfalls noch ein vorteilhafter Drall in Verbindung mit einer Vertikalströmung erzielen, da dann im Bereich der Gehäuseöffnungen unterschiedlich gerichtete Ströme austreten.

Je nach Flüssigkeitsspiegel oder auch je nach gewünschter Betriebsart ist die Schnecke vorzugsweise höhenverstellbar ausgebildet, so dass vorgesehen werden kann, dass beide Gehäuseöffnungen unterhalb der Flüssigkeitsoberfläche in die Fermenterflüssigkeit eingetaucht sind oder aber nur eine der beiden Gehäuseöffnungen, nämlich die untere in die Fermenterflüssigkeit eingetaucht ist. Dadurch kann die Rühreinrichtung individuell zwischen den unterschiedlichen zuvor geschilderten Betriebsarten verstellt werden.

Ferner ist mit einem derartigen Aufbau auch eine einfache Verschwenkung der Rühreinrichtung um eine Schneckengehäuselängsachse möglich, da z. B. lediglich der Antriebsstrang durch eine Behälterseitenwand dicht und verdrehbar durchzuführen ist, wobei vorzugsweise eine lösbare Festlegung und/oder Verrastung in unterschiedlichen Schwenkstellungen vorgesehen ist. Dadurch kann z. B. auf lokale Feststoffansammlungen in der Fermenterflüssigkeit noch gezielter reagiert werden.

Zudem können im Bereich der Gehäuseöffnungen auch noch Strömungsleitbleche vorgesehen sein, die ebenfalls eine gerichtete und gezielte Ausbildung der Strömung ermöglichen.

Gemäß einer besonders bevorzugten Ausführungsform ist das Schneckengehäuse an einem Seitenwandbereich des Fermenterbehälters festlegbar, vorzugsweise dort höhenverstellbar festlegbar, wobei ein unteres Gehäuseende in einem vorgebbaren Abstand zum Fermenterbehälterboden angeordnet ist. Eine derartige Festlegung des Schneckengehäuses am Seitenwandbereich ist besonders einfach zu bewerkstelligen und beeinträchtigt die Rührergebnisse nicht, da sich die Vertikalströmung sehr gut über die gesamte Fermenterflüssigkeit bis in den gegenüberliegenden Seitenwandbereich ausdehnt. Der Antriebsstrang kann dann beispielsweise im seitlichen Fermenterbehälterdeckenbereich durchgeführt sein, so dass sich auch für eine Wartung eine besonders einfache Zugänglichkeit ergibt.

Gemäß einer besonders vorteilhaften Doppelfunktion ist die durch die Schnecke und das Schneckengehäuse gebildete Rühreinrichtung zudem als Biomasse-Beschickungseinrichtung ausgebildet. Ein Gehäuseendbereich des Schneckengehäuses ist hierzu bis in einen Bereich außerhalb des Fermenterbehälters führbar für eine Beschickung mit vorzugsweise Biomasse-Feststoffen, die mittels der Schnecke bei deren Antrieb in die Vorwärtsrichtung in die Fermenterflüssigkeit eingedrückt werden können. Bevorzugt erfolgt hierbei die Durchführung des Gehäuses durch eine Fermenterbehälterdecke des Fermenterbehälters. Zur Zuführung von Biomasse-Feststoffen ist hierbei vorzugsweise am Beschickungs-Gehäuseendbereich ein Einwurftrichter vorgesehen, in dessen Bereich die Schnecke geführt ist. Dadurch ist sichergestellt, dass die Biomasse-Feststoffe mittels der Schnecke schnell abtransportiert werden kann, so dass eine effektive Zudosierung auch von größeren Mengen möglich ist.

Gemäß einer konkreten Ausgestaltung hierzu kann die erste, obere Gehäuseöffnung in einem Seitenwandbereich des einen Rohrquerschnitt aufweisenden und im Fermenterbehälterinnenraum angeordneten Schneckengehäusebereiches ausgebildet sein, während die zweite, untere Gehäuseöffnung in einem Seitenwandbereich des Schneckengehäuses oder besonders bevorzugt durch die untere Rohröffnung ausgebildet ist. Bevorzugt sind hierzu die im Seitenwandbereich ausgebildeten Gehäuseöffnungen mit einem Verschlusselement dicht verschließbar, so dass der Feststoffeintrag über die Schnecke nicht beeinträchtigt wird. Dies kann z. B. dann der Fall sein, wenn die Gehäuseöffnungen relativ groß ausgebildet sind. Als Verschlusselement kann z. B. ein einfacher Schieber vorgesehen sein, z. B. ein mechanischer Schieber, der manuell betätigbar ist. Es können jedoch auch elektrische und/oder pneumatische und/oder hydraulische Schieberanordnungen bzw. Verschlussanordnungen vorgesehen sein, die jedoch insgesamt aufwendiger sind.

Gemäß eine weiteren besonders bevorzugten Ausgestaltung der Erfindung kann der Fermenterbehälter mittels einem Überlaufrohr mit einem benachbarten Fermenterbehälter strömungsverbunden sein, wobei das Überlaufrohr mit einem ersten Überlaufrohrende in einer mittels einem Ausnehmungs-Verschlusselement, vorzugsweise einem Schieber, verschließbaren Gehäuseausnehmung des Schneckengehäuses mündet und mit einem zweiten Überlaufrohrende durch eine Fermenterbehälterwand des zweiten Fermenterbehälters in dessen Fermenterbehälterinnenraum geführt ist. Dadurch wird erreicht, dass bei geöffnetem Ausnehmungs-Verschlusselement mittels der Schnecke und einem entsprechendem Antrieb die mit Biomasse-Feststoffen versetzte Fermenterflüssigkeit aus dem ersten Fermenterbehälter über das Überlaufrohr in den zweiten Fermenterbehälter gepumpt werden kann. Das Überlaufrohr weist dabei vorzugsweise in Fließrichtung ein Gefälle auf.

Vorteilhaft ergibt sich somit bei einem derartigen Aufbau eine Dreifachfunktion der Schnecke mitsamt Schneckengehäuse, nämlich einmal als Rühreinrichtung in der zuvor beschriebenen Art und Weise, dann in einer zweiten Funktion als Beschickungseinrichtung zum Eindrücken von insbesondere Biomasse-Feststoffen in die Fermenterflüssigkeit und zum Dritten eine Pumpenfunktion, da mittels der Schnecke dann auch Fermenterflüssigkeit in einen zweiten benachbarten Fermenterbehälter gepumpt werden kann.

Im Pumpbetrieb ist dabei vorzugsweise die im Seitenwandbereich ausgebildete Gehäuseöffnung mittels dem Gehäuseöffnungs-Verschlusselement verschlossen, so dass ein Einströmen der Fermenterflüssigkeit in das Schneckengehäuse in eine untere Rohröffnung des Schneckengehäuses als untere Gehäuseöffnung erfolgt. Dadurch ergibt sich ein vorteilhaft einfacher Aufbau insgesamt. Die Gehäuseausnehmung ist dabei im Nicht-Pumpbetrieb mit dem Ausnehmungs-Verschlusselement verschlossen, um die beiden anderen Funktionen, nämlich die Pumpfunktion und die Rührfunktion der Rühreinrichtung nicht zu beeinträchtigen.

Ferner kann eine Steuer- und Regeleinrichtung vorgesehen sein, mittels der der Schneckenantrieb und/oder die einzelnen Verschlusselemente entsprechend dem gewünschten, anwählbaren Betriebszustand ansteuerbar sind. Mit einer derartigen Steuer- und Regeleinrichtung ist ein vorteilhafter automatisierter Betrieb der Rühreinrichtung möglich. Ebenso kann hierdurch z.B. die Drehzahl der Schnecke vorteilhaft reguliert werden, um die Drehzahl variabel an das jeweilige Medium anzupassen. So muss beispielsweise im praktischen Betrieb die Drehzahl bei dickflüssigeren Medien langsamer sein als bei dünnflüssigeren Medien.

Grundsätzlich können auch mehrere derartiger Rühreinrichtungen in einem Fermenterbehälter vorgesehen sein.

Die Erfindung wird nachfolgend anhand einer Zeichnung näher erläutert.

Es zeigen:
- Fig. 1: eine schematische Seitenansicht einer Prinzipdarstellung einer in einem Fermenterbehälterinnenraum angeordneten erfindungsgemäßen Rühreinrichtung,
- Fig. 2: eine schematische perspektivische Darstellung der Anordnung gemäß Fig. 1,
- Fig. 3: eine schematische Seitenansicht einer Prinzipdarstellung einer an einem Seitenwandbereich angeordneten Rühreinrichtung,
- Fig. 4: eine schematische perspektivische Ansicht der Darstellung gemäß Fig. 3,
- Fig. 5: eine schematische Seitenansicht entsprechend Fig. 3 mit im unteren Schneckengehäusebereich angeordnetem Auslaufstutzen,
- Fig. 6: eine schematische Draufsicht auf die Darstellung gemäß Fig. 5,
- Fig. 7: eine vergrößerte, schematische Detailansicht eines mehrfach verzweigten Auslaufstutzens,
- Fig. 8: eine konkrete, schematische Seitenschnittansicht durch eine Biogasanlage mit zwei Fermenterbehältern und einer Überlaufanordnung vom ersten Fermenterbehälter in den zweiten Fermenterbehälter,
- Fig. 9: eine alternative Ausführungsform zum Aufbau gemäß Fig. 8, und
- Fig. 10 bis 12: unterschiedliche Schieberstellungen für die unterschiedlichen Funktionen der erfindungsgemäßen Anordnung gemäß Fig. 9.

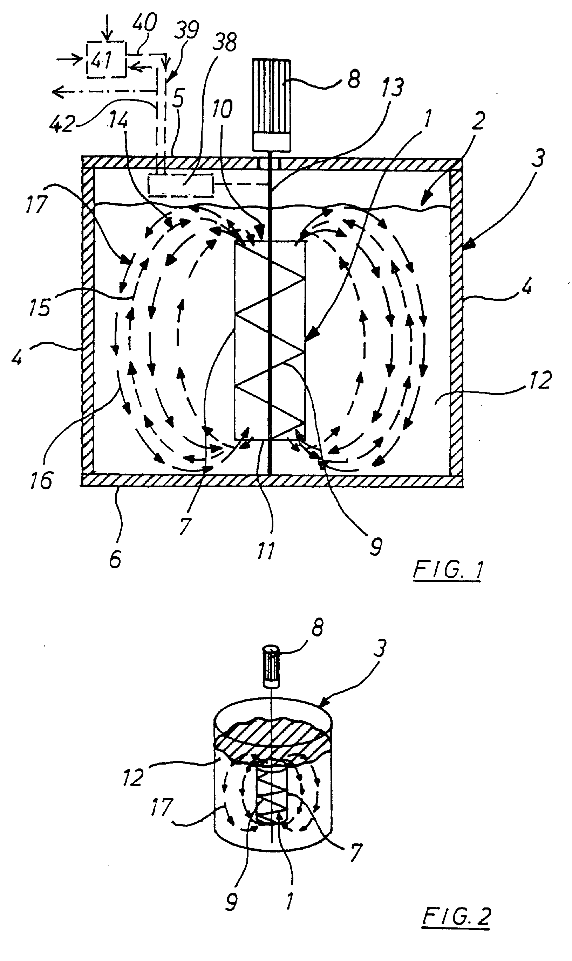

In Fig. 1 ist schematisch eine Seitenansicht als Prinzipdarstellung zur Erklärung der erfindungsgemäßen Rühreinrichtung 1 in einem Fermenterbehälterinnenraum 2 eines Fermenterbehälters 3 gezeigt. In der Darstellung der Fig. 2 ist eine schematische perspektivische Darstellung gezeigt.

Wie dies den beiden Figuren zu entnehmen ist, weist der Fermenterbehälter 3 eine Fermenterbehälterseitenwand 4, eine Fermenterbehälterdecke 5 und einen Fermenterbehälterboden 6 auf.

Die Rühreinrichtung 1 ist hier durch eine in einem rohrförmigen Schneckengehäuse 7 aufgenommene und mittels einem außerhalb des Fermenterbehälters 3 angeordneten Schneckenantrieb 8, z. B. einem Elektromotor, in eine Vorwärtsrichtung und in eine Rückwärtsrichtung antreibbare Schnecke 9 gebildet. Alternativ zu einem außerhalb des Fermenterbehälters 3 angeordneten Schneckenantrieb 8 kann auch, wie dies in der Fig. 1 lediglich äußerst schematisch und strichliert dargestellt ist, der Schneckenantrieb 38 als Hydraulikmotor oder Pneumatikmotor ausgebildet sein, der mittels einer Hydraulikflüssigkeit oder Luft antreibbar ist. Dieser Schneckenantrieb 38 ist mit einer Energieleitung 39 verbunden. Diese Energieleitung 39 weist eine Rohr- und/oder Schlauchleitung als Zuführleitung 40 für das Medium, die an eine Medium-Kompressionseinheit 41, entweder ein Hydraulik-Kompressor oder ein Luftkompressor, angeschlossen ist und dicht durch die Fermenterbehälterdecke 5 als Fermenterbehälterwand zum Schneckenantrieb 38 hin geführt ist. Weiter umfasst diese Energieleitung 39 eine Rohr- und/oder Schlauchleitung als Abführleitung 42 für das Medium, die ebenfalls dicht durch die Fermenterbehälterdecke aus dem Fermenterbehälter herausgeführt ist. Die Medium-Kompressionseinheit 41 ist hier elektrisch betreibbar und außerhalb des Fermenterbehälters 3 angeordnet, so dass im Fermenterbehälterinnenraum 2 eine explosionsgeschützte Anordnung des Schneckenantriebs 38, z.B. im Bereich der Gasphase, möglich ist. Für den Fall, dass Luft als Arbeitsmedium verwendet wird, braucht die Abführleitung 42, wie dies in der Darstellung der Fig. 1 lediglich ganz schematisch und beispielhaft strichpunktiert dargestellt ist, nicht wieder zum dann Luftkompressor als Medium-Kompressionseinheit 41 zurückgeführt werden, sondern kann auch in die Umgebung abblasen. Für den Fall der Verwendung eines Inertgases oder einer Hydraulikflüssigkeit ist dagegen die Abführleitung 42, wie dies in der Fig. 1 strichliert dargestellt ist, als Rückleitung in einem geschlossenen Kreislauf an die Medium-Kompressionseinheit 41 angeschlossen.

Ein oberes Rohröffnungsende 10 bildet hier eine erste, obere Gehäuseöffnung, während eine davon in Gehäuselängsrichtung beabstandete, zweite Gehäuseöffnung durch ein unteres Rohröffnungsende 11 gebildet ist. Das gesamte Schneckengehäuse 7 ist hier vollständig in eine mit Biomasse-Feststoffen versetzte Fermenterflüssigkeit 12 eingetaucht.

Die Rühreinrichtung 1 ist im Fermenterbehälterinnenraum 2 am Fermenterbehälterboden 6 gelagert, was hier jedoch nicht im Detail dargestellt ist. Zudem ist ein Antriebstrang 13 des Schneckenantriebs hier gasdicht durch die Fermenterbehälter-Decke 5 durchgeführt, was hier jedoch ebenfalls nicht im Detail dargestellt ist.

Beim Antrieb der Schnecke 9 mittels dem Schneckenantrieb 8 in eine Vorwärtsrichtung strömt über das obere Rohröffnungsende 10 entsprechend den strichlierten Pfeilen 15 der Fig. 1 ein mit Biomasse-Feststoffen versetzter Fermenterflüssigkeitsanteil in das Schneckengehäuse 7 ein, so dass anschließend die Schnecke 9 diesen mit Biomasse-Feststoffen versetzten Fermenterflüssigkeitsanteil ggf. unter Zerkleinerung der Biomasse-Feststoffe in Richtung zum unteren Rohröffnungsende 11 fördert, über das mit Biomasse-Feststoffen versetzte Fermenterflüssigkeitsanteil entsprechend den strichlierten Pfeilen 15 in Fig. 1 im Kreislauf wieder aus dem Schneckengehäuse heraus in die Fermenterflüssigkeit 12 rückströmt, so dass sich um den Schneckengehäuseumfang herum eine Vertikalströmung 14 in der Fermenterflüssigkeit 12 ausbildet. Diese bewirkt, dass sich z. B. an der Flüssigkeitsoberfläche ansammelnde Biomasse-Feststoffe, d. h. sogenannte Schwimmschichten, durch die Vertikalströmung 14 gleichmäßig in der Fermenterflüssigkeit 12 verteilen und zwar einmal über die Rühreinrichtung 1 als solche und zum anderen über die sich in der Fementerflüssigkeit 12 ausbildende Strömung.

Im umgekehrten Fall bei einem Antrieb der Schnecke 9 in die Rückwärtsrichtung wird entsprechend der mit durchgezogenen Linien gezeichneten Pfeile 15 ein mit Biomasse-Feststoffen versetzter Fermenterflüssigkeitsanteil über das untere Rohröffnungsende 11 angesaugt. Dieser wird dann mittels der Schnecke 9 in den Bereich des oberen Rohröffnungsendes 10 gefördert und dort wieder im Kreislauf in die Fermenterflüssigkeit eingetragen. Dadurch bildet sich die in der Fig. 1 dargestellte Vertikalströmung 14 um den Schneckengehäuseumfang herum aus, die anders als die Vertikalströmung 17 in Gegenuhrzeigerrichtung verläuft. Mit einer derartigen Vertikalströmung 17 können insbesondere bodennahe Sinkschichten von Biomasse-Feststoffen für eine gleichmäßige Verteilung in den Bereich des oberen Flüssigkeitsspiegels umverteilt werden. Auch dies wird durch die Schneckenanordnung noch zusätzlich unterstützt.

Dadurch ergibt sich eine gleichmäßige Verteilung der Biomasse-Feststoffe in der Fermenterflüssigkeit, was wiederum für einen hohen Wirkungsgrad der Fermentation insgesamt von Vorteil ist. Durch entsprechendes Umschalten zwischen Vorwärts- und Rückwärtsrichtung kann dabei einfachst auf die jeweiligen Erfordernisse abgestellt werden.

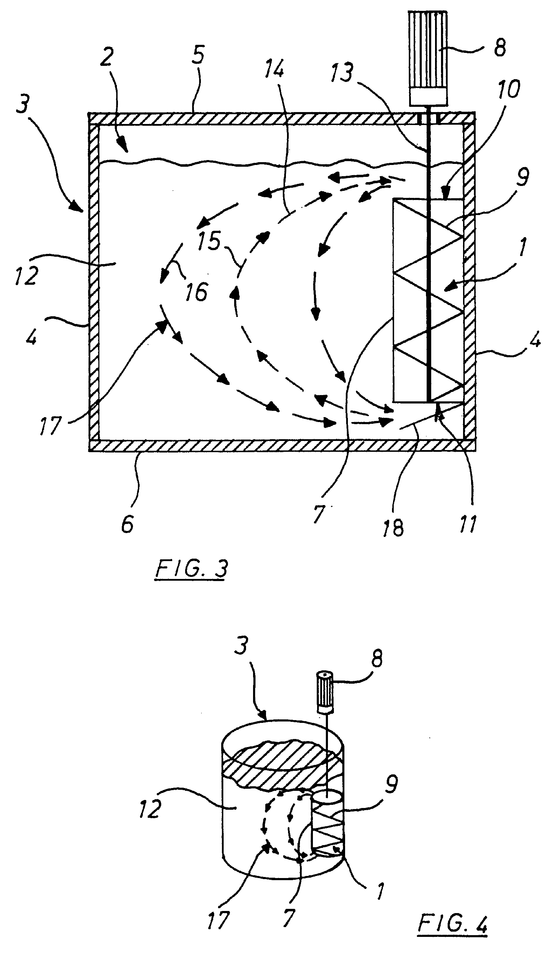

In den Fig. 3 und 4 ist ein vom Prinzip her der Fig. 1 ähnlicher Aufbau gezeigt, so dass hier auf die Ausbildung der Vertikalströmungen nicht mehr näher eingegangen wird. Im Unterschied zu der Ausführungsform gemäß den Fig. 1 und 2 ist hier jedoch das Schneckengehäuse 7 am Fermenterbehälter-Seitenrandbereich 4 angeordnet. Ebenso wie bei der Ausführungsform gemäß Fig. 1 kann hier eine Höhenverstellung vorgesehen sein. Im Bereich des unteren Rohröffnungsendes 11 ist ferner ein Strömungsleitblech 18 vorgesehen, mit dem eine gezielte Strömungsrichtung in diesem Bereich vorgegeben werden kann. Ggf. könnte ein derartiges Strömungsleitblech 18 zusätzlich oder alternativ auch im Bereich der oberen Gehäuseöffnung als oberes Rohröffnungsende 10 angeordnet sein.

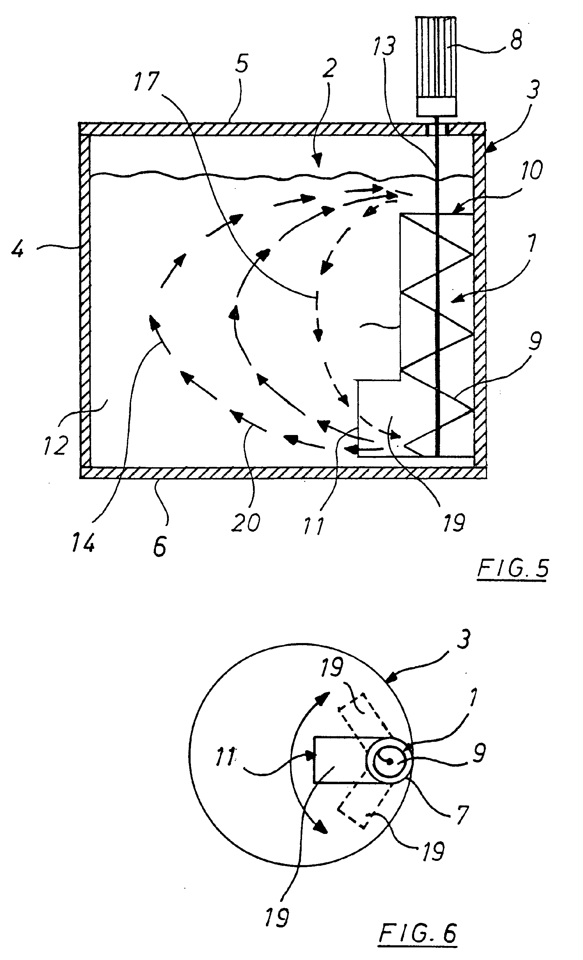

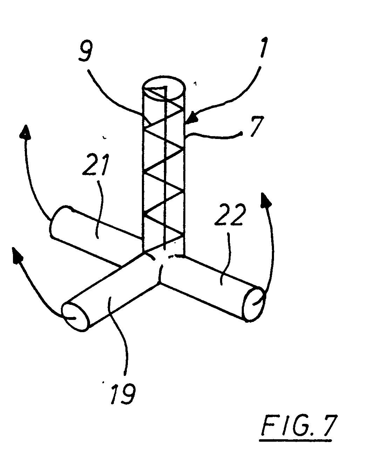

In der Fig. 5 ist ein vom Prinzip her ähnlicher Aufbau wie in den Fig. 3 und 4 gezeigt, wobei im Bereich des unteren Rohröffnungsendes 11 ein gegenüber der Schneckengehäuselängsrichtung hier beispielhaft um 90° abgewinkelter Auslaufstutzen 19 vorgesehen ist. Ein derartiger Auslaufstutzen 19 bewirkt, wie dies in der Fig. 5 durch die Pfeile 20 dargestellt ist, ein besonders vorteilhaftes Vertikalströmungsprofil, da durch diesen Auslaufstutzen 19 der angesaugte und vorzugsweise mit Biomasse-Feststoffen versetzte Fermenterflüssigkeitsanteil hierdurch sehr einfach an die gegenüberliegende Wand gespült werden kann, dort hochsteigt und dann wieder im Kreislauf von oben am oberen Rohröffnungsende 10 angesaugt wird. Dies stellt eine komplette Durchmischung sicher. Eine komplette Durchmischung kann dabei auch dadurch erreicht werden, dass das Schneckengehäuse 7 seitlich verschwenkt wird, wie dies aus der Draufsicht der Fig. 6 ersichtlich ist. Grundsätzlich wäre es hier auch denkbar, gegenüberliegend in unterschiedlichen Höhen entsprechende Rühreinrichtungen 1 anzubringen. Gemäß einer weiteren Ausgestaltung hierzu kann, wie dies in der Fig. 7 dargestellt ist, der Auslaufstutzen mehrfach verzweigt sein, vorzugsweise so, dass mehrere Auslaufstutzen 19, 21, 22 in einer Auslaufstutzen-Horizontalebene liegen, die voneinander mit vorgebbaren Winkelabständen beabstandet sind. Dies ermöglicht eine vorteilhafte Durchmischung der Fermenterflüssigkeit 12 mit Biomasse-Feststoffen.

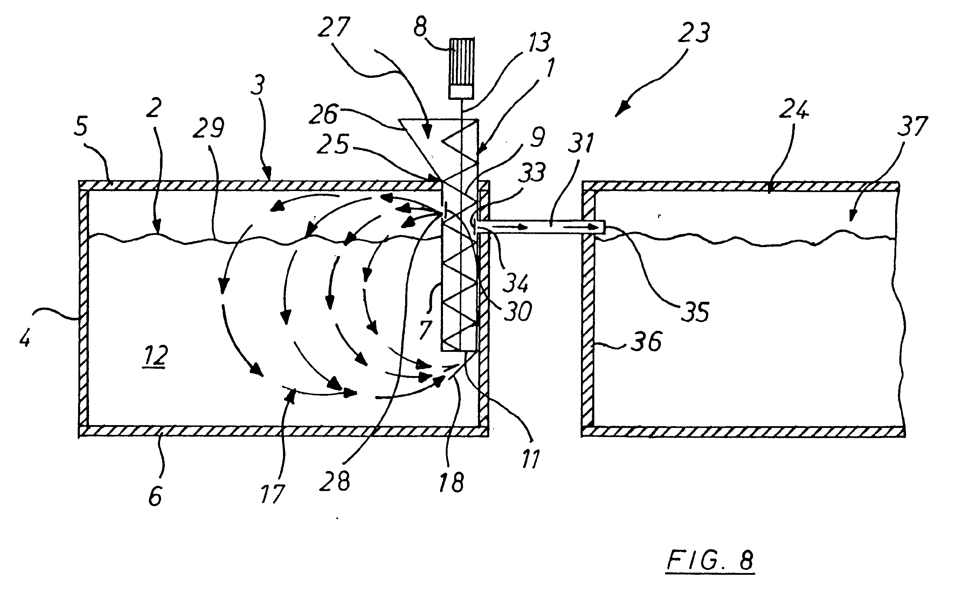

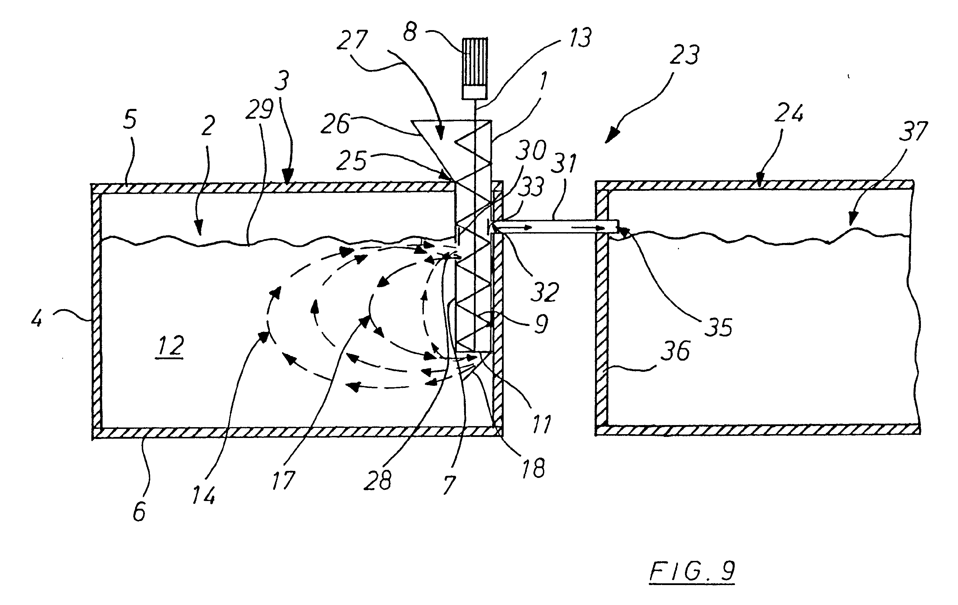

In den Fig. 8 und 9 ist ein bevorzugter Einsatzfall der erfindungsgemäßen Rühreinrichtung 1 in einer Biogasanlage 23 gezeigt, die zusätzlich zu dem ersten Fermenterbehälter 3 noch einen zweiten Fermenterbehälter 24 aufweist. Die Rühreinrichtung 1 ist hier in der zuvor bereits in Verbindung mit den Fig. 3 und 4 beschriebenen Weise an einer Fermenterbehälter-Seitenwand 4 festgelegt und zugleich als Biomasse-Beschickungseinrichtung ausgebildet. Dazu ist ein Gehäuseendbereich 25 des Schneckengehäuses 7 durch die Fermenterbehälter-Decke 5 hindurch nach außerhalb des Fermenterbehälters 3 geführt, wobei auf diesen Gehäuseendbereich 25 ein Einwurftrichter 26 aufgesetzt ist oder integral mit diesem verbunden ist. Die Schnecke 9 ist hier bis in den Bereich des Einwurftrichters 26 geführt. Über diesen Einwurftrichter 26 können dann entsprechend dem Pfeil 27 Biomasse-Feststoffe zu der Rühreinrichtung 1 zugeführt werden, die dann mittels der Schnecke 9 bei deren Antrieb in die Vorwärtsrichtung in die Fermenterflüssigkeit 12 über das untere Rohröffnungsende 11 eingedrückt werden.

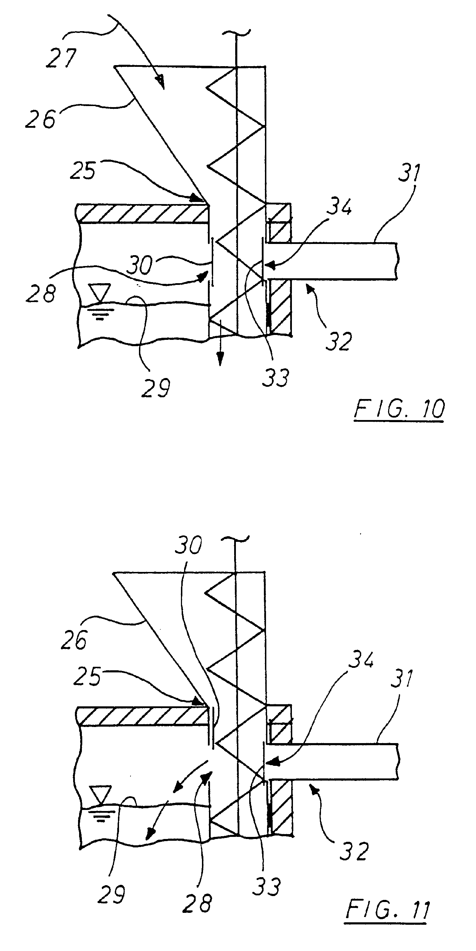

Wie dies der Fig. 8 ferner entnommen werden kann, ist an einem Seitenwandbereich des Schneckengehäuses 7 eine obere Gehäuseöffnung 28 ausgebildet, die im in der Fig. 8 gezeigten Aufbau oberhalb des Flüssigkeitsspiegels 29 der Fermenterflüssigkeit 12, d. h. in der Gasphase angeordnet ist. Diese obere Gehäuseöffnung 28 ist, wie dies insbesondere aus den Fig. 10 bis 12 ersichtlich ist, mittels einem Schieber 30 verschließbar.

Wie dies weiter aus Fig. 8 ersichtlich ist, ist der erste Fermenterbehälter 3 mittels einem Überlaufrohr 31 mit dem zweiten Fermenterbehälter 24 strömungsverbunden, wobei das Überlaufrohr 31 mit einem ersten Überlaufrohrende 32 in einer mittels einem Schieber 33 verschließbaren Gehäuseausnehmung 34 des Schneckengehäuses 7 mündet, wie dies wiederum insbesondere aus den Fig. 10 bis 12 ersichtlich ist, die eine vergrößerte Detailansicht dieses Bereichs zeigen. Auch andere Verschließmaßnahmen sind hier denkbar und möglich.

Ein zweites Überlaufrohrende 35 ist durch eine Fermenterbehälter-Seitenwand 36 des zweiten Fermenterbehälters 24 in dessen Fermenterbehälterinnenraum 37 geführt.

Im in der Fig. 10 gezeigten Zustand der Schieber 30, 33 kann über den Einwurftrichter 26 Biomasse zugeführt und in die Fermenterflüssigkeit 12 eingedrückt werden, wobei durch die geschlossenen Schieber 30, 33 sichergestellt ist, dass keine Biomasse in das Überlaufrohr 31 gelangen bzw. über die obere Gehäuseöffnung 28 auf die Fermenterbehälter Flüssigkeitsoberfläche 29 gelangen kann. Die Rühreinrichtung 1 hat hier somit eine Funktion als Biomasse-Beschickungseinrichtung.

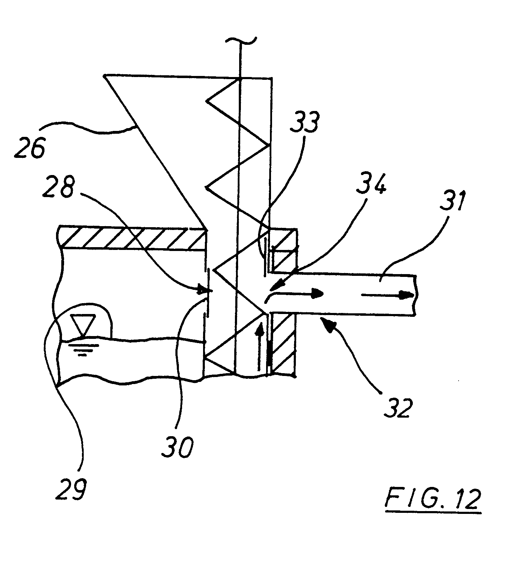

Für den in Verbindung mit den Fig. 1 bis 2 ausführlich beschriebenen Rührbetrieb der Rühreinrichtung 1 kann dann, wie dies in der Fig. 11 beispielhaft dargestellt ist, der Schieber 30 geöffnet werden, so dass die obere Gehäuseöffnung 28 freigegeben ist. Beim Antrieb der Schnecke 9 in die Rückwärtsrichtung kann dann, wie dies auch schematisch aus der Fig. 8 ersichtlich ist, über das untere Rohröffnungsende 11 mit Biomasse-Feststoffen versetzte Fermenterflüssigkeiten angesaugt werden und anschließend über die obere Gehäuseöffnung 28 wieder aus dem Schneckengehäuse 7 heraus in den Fermenterbehälterinnenbehälter 2 rückströmen. Dadurch ist eine besonders vorteilhafte gute Vermischung und Verteilung der Biomasse-Feststoffe in der Fermenterflüssigkeit 12 durch Ausbildung einer Vertikalströmung möglich.

Wie dies aus der Fig. 12 weiter zu entnehmen ist, die die Schieberstellung im Pumpbetrieb der Rühreinrichtung 1 zeigt, ist der Schieber 30 verschlossen, so dass bei einem Rückwärtsantrieb der Schnecke 9 bei geöffnetem Schieber 33 über das Überlaufrohr 31 Fermenterflüssigkeit 12 in den zweiten Fermenterbehälter 24 gepumpt werden kann.

In der Fig. 9 ist eine alternative Ausführungsform des Aufbaus gemäß Fig. 8 gezeigt, bei der die obere Gehäuseöffnung 28 nicht im Bereich der Gasphase liegt, somit im Bereich der Flüssigkeitsphase, d. h. unterhalb des Flüssigkeitsspiegels der Fermenterflüssigkeit angeordnet und in die Fermenterflüssigkeit 12 eingetaucht ist. Wie in der Fig. 9 durch die strichlierten und durchgezogenen Pfeile 15, 16 gezeigt, kann dann hier eine sowohl im als auch gegen den Uhrzeigersinn gerichtete Vertikalströmung sehr gut ausgebildet werden.

Durch entsprechende Maßnahmen im Bereich der Gehäuseöffnungen bzw. Rohröffnungsenden, z. B. dem Anbringen von Auslaufstutzen, Strömungsleitblechen oder dergleichen, kann hier ohne weiteres eine Vertikalströmung ausgebildet werden, die sich bei einer Anordnung der Rühreinrichtung 1 an einem Fermenterbehälter-Seitenwandbereich vollständig über die gesamte Fermenterflüssigkeit 12 ausbildet. Dies ist in den schematischen Prinzipdarstellungen der Fig. 8 und 9 aus Übersichtlichkeitsgründen jedoch weggelassen worden.

## Patentansprüche

1. Rühreinrichtung für einen Fermenter einer Biogasanlage, die in einem Fermenterbehälterinnenraum eines Fermenterbehälters zum Umwälzen einer insbesondere mit Biomasse-Feststoffen versetzten Fermenterflüssigkeit anordenbar ist,
**dadurch gekennzeichnet,**
**dass** die Rühreinrichtung (1) durch eine in einem Schneckengehäuse (7) aufgenommene und mittels einem Schneckenantrieb (8; 38) in eine Vorwärtsrichtung und/oder eine Rückwärtsrichtung antreibbare Schnecke (9) gebildet ist,
**dass** am Schneckengehäuse (7) eine erste Gehäuseöffnung (10; 28) und eine davon in Gehäuselängsrichtung beabstandete, zweite Gehäuseöffnung (11) vorgesehen ist, und
**dass** wenigstens eine der beiden Gehäuseöffnungen (10, 11; 28) im montierten Zustand der Rühreinrichtung (1) in die mit Biomasse-Feststoffen (12) versetzte Fermenterflüssigkeit eintaucht.

2. Rühreinrichtung nach Anspruch 1, **dadurch gekennzeichnet,**
**dass** im montierten Zustand der Rühreinrichtung (1) die zweite Gehäuseöffnung (11) als untere Gehäuseöffnung in die Fermenterflüssigkeit (12) eingetaucht ist und die erste Gehäuseöffnung (28) als obere Gehäuseöffnung im Gasphasenbereich oberhalb des Flüssigkeitsspiegels (29), vorzugsweise unmittelbar oberhalb des Flüssigkeitsspiegels (29), angeordnet ist dergestalt,
**dass** bei einem Antrieb der Schnecke mittels dem Schneckenantrieb (8; 38) in eine Rückwärtsrichtung über die untere Gehäuseöffnung (11) ein Fermenterflüssigkeitsanteil mitsamt Biomasse-Feststoffen im Kreislauf in das Schneckengehäuse (7) einströmt und mittels der Schnecke (9) in den Bereich der oberen Gehäuseöffnung (28) förderbar ist, über die der mit Biomasse-Feststoffen versetzte Fermenterflüssigkeitsanteil wieder aus dem Schneckengehäuse (7) ausströmt zur Zuführung zur Fermenterflüssigkeit (12) im Fermenterbehälterinnenraum (2) zur Ausbildung einer bezogen auf das Schneckengehäuse (7) Vertikalströmung (17) als Tumbleströmung in der Fermenterflüssigkeit (12).

3. Rühreinrichtung nach Anspruch 1, **dadurch gekennzeichnet,**
**dass** beide Gehäuseöffnungen (10, 11; 28) im montierten Zustand der Rühreinrichtung (1) in die Fermenterflüssigkeit (12) eintauchen,
**dass** bei einem Antrieb der Schnecke (9) in die Vorwärtsrichtung über die obere Gehäuseöffnung (10; 28) ein mit Biomasse-Feststoffen versetzter Fermenterflüssigkeitsanteil im Kreislauf in das Schneckengehäuse (7) einströmt und mittels der Schnecke (9) in den Bereich der unteren Gehäuseöffnung (11) förderbar ist, über die der mit Biomasse-Feststoffen versetzte Fermenterflüssigkeitsanteil wieder aus dem Schneckengehäuse (7) heraus in die Fermenterflüssigkeit (12) im Fermenterbehälterinnenraum (2) rückströmt zur Ausbildung einer bezogen auf das Schneckengehäuse (7) Vertikalströmung (14) als Tumbleströmung in der Fermenterflüssigkeit (12), und/oder
**dass** bei einem Antrieb der Schnecke mittels dem Schneckenantrieb (8; 38) in eine Rückwärtsrichtung über die untere Gehäuseöffnung (11) ein Fermenterflüssigkeitsanteil mitsamt Biomasse-Feststoffen im Kreislauf in das Schneckengehäuse (7) einströmt und mittels der Schnecke (9) in den Bereich der oberen Gehäuseöffnung (28) förderbar ist, über die der mit Biomasse-Feststoffen versetzte Fermenterflüssigkeitsanteil wieder aus dem Schneckengehäuse (7) ausströmt zur Zuführung zur Fermenterflüssigkeit (12) im Fermenterbehälterinnenraum (2) zur Ausbildung einer bezogen auf das Schneckengehäuse (7) Vertikalströmung (17) als Tumbleströmung in der Fermenterflüssigkeit (12).

4. Rühreinrichtung nach einem der Ansprüche 1 bis 3, **dadurch gekennzeichnet, dass** die Schnecke (9) mitsamt Schneckengehäuse (7) im montierten Zustand in etwa vertikal ausgerichtet im Fermenterbehälterinnenraum (2) angeordnet ist.

5. Rühreinrichtung nach einem der Ansprüche 1 bis 4, **dadurch gekennzeichnet, dass** der Schneckenantrieb (8) außerhalb des Fermenterbehälters (3) angeordnet ist, vorzugsweise ein Antriebsstrang (13) des Schneckenantriebs (8) dicht durch eine Fermenterbehälterdecke (5) des Fermenterbehälters (3) geführt ist.

6. Rühreinrichtung nach einem der Ansprüche 1 bis 5, **dadurch gekennzeichnet,**
**dass** der Schneckenantrieb (38) im Fermenterbehälter (3) angeordnet ist und mittels einem Arbeitsmedium antreibbar ist,
**dass** der Schneckenantrieb (38) mit einer Energieleitung (39) verbunden ist,
**dass** die Energieleitung (39) einerseits eine Rohr- und/oder Schlauchleitung als Zuführleitung (40) für das Medium, die an eine Medium-Kompressionseinheit (41) angeschlossen ist und die dicht durch eine Behälterwand (5) zum Schneckenantrieb (38) geführt ist, und andererseits eine Rohr- und/oder Schlauchleitung als Abführleitung (42) für das Medium, die dicht durch eine Behälterwand (5) aus dem Fermenterbehälter (3) herausgeführt ist, umfasst, und
**dass** die Medium-Kompressionseinheit (41) elektrisch betreibbar ist und außerhalb des Fermenterbehälters (3) angeordnet ist.

7. Rühreinrichtung nach Anspruch 6, **dadurch gekennzeichnet,**
**dass** der Schneckenantrieb (38) ein Hydraulik-Motor ist, wobei das Medium eine Hydraulikflüssigkeit ist und die Medium-Kompressionseinheit (41) ein Hydraulik-Kompressor ist, und
**dass** die Abführleitung (42) als Rückleitung in einem geschlossenen Hydraulikkreislauf an den Hydraulik-Kompressor angeschlossen ist.

8. Rühreinrichtung nach Anspruch 6, **dadurch gekennzeichnet,**
**dass** der Schneckenantrieb (38) ein Pneumatik-Motor ist, wobei das Medium Luft ist und die Medium-Kompressionseinheit (41) ein Luftkompressor ist, und
**dass** die Abführleitung (42) als Abblasleitung dicht aus dem Fermenterbehälter geführt ist.

9. Rühreinrichtung nach einem der Ansprüche 1 bis 8, **dadurch gekennzeichnet, dass** das Schneckengehäuse (7) an einem Seitenwandbereich des Fermenterbehälters (3) festgelegt ist, vorzugsweise mit einem unteren Gehäuseende in einem vorgebbaren Abstand zum Fermenterbehälterboden angeordnet ist.

10. Rühreinrichtung nach einem der Ansprüche 1 bis 9, **dadurch gekennzeichnet,**
**dass** im Bereich der in die Fermenterflüssigkeit (12) eintauchenden Gehäuseöffnungen (10, 11; 28), vorzugsweise an einem oder beiden Schneckengehäuseenden, ein gegenüber der Schneckengehäuselängsrichtung abgewinkelter Auslaufstutzen (19), vorzugsweise ein bei vertikaler Schneckenausrichtung um 90° abgewinkelter Auslaufstutzen, anordenbar oder integral damit ausgebildet ist.

11. Rühreinrichtung nach Anspruch 10, **dadurch gekennzeichnet, dass** mehrere derartiger Auslaufstutzen (19, 21, 22) in einer Auslaufstutzen-Horizontalebene liegend vorgesehen sind, die voneinander mit vorgebbaren Winkelabständen beabstandet sind.

12. Rühreinrichtung nach einem der Ansprüche 1 bis 11, **dadurch gekennzeichnet, dass** die Rühreinrichtung (1) höhenverstellbar ist und/oder um eine Schneckengehäuselängsachse schwenkbar ist, vorzugsweise in unterschiedlichen Schwenkstellungen lösbar festlegbar und/oder verrastbar ist.

13. Rühreinrichtung nach einem der Ansprüche 1 bis 12, **dadurch gekennzeichnet, dass** im Bereich der Gehäuseöffnung (11) wenigstens ein Strömungsleitblech (18) anordenbar ist.

14. Rühreinrichtung nach einem der Ansprüche 1 bis 13, **dadurch gekennzeichnet, dass** das Schneckengehäuse (7) einen Rohrquerschnitt, vorzugsweise einen eckigen oder ovalen Querschnitt, aufweist, und
dass wenigstens eine der beiden Gehäuseöffnungen (10, 11; 28) durch eine endseitige Rohröffnung (10) des Schneckengehäuses (7) und/oder in einem Seitenwandbereich des Schneckengehäuses (7) ausgebildet ist.

15. Rühreinrichtung nach einem der Ansprüche 1 bis 14, **dadurch gekennzeichnet,**
**dass** die Schnecke (9) formschlüssig im Schneckengehäuse (7) aufgenommen ist, und/oder
**dass** die Wendelflächen, bevorzugt die Außenkanten, der Schnecke (9) wenigstens teilweise aus einem harten Material hergestellt sind, und/oder
**dass** die Schnecke (9) durch eine Schneckenwelle und/oder eine Schraubenfläche gebildet ist.

16. Rühreinrichtung nach einem der Ansprüche 1 bis 15, **dadurch gekennzeichnet,**
**dass** die Rühreinrichtung (1) in einer Doppelfunktion als Biomasse-Beschickungseinrichtung ausgebildet ist dergestalt,
**dass** ein Beschickungs-Gehäuseendbereich des Schneckengehäuses (7) bis in einen Bereich außerhalb des Fermenterbehälters (3) führbar, vorzugsweise durch eine Fermenterbehälterdecke (5) des Fermenterbehälters (3) geführt ist, für eine Beschickung mit Biomasse-Feststoffen, die mittels der Schnecke (9) bei deren Antrieb in die Vorwärtsrichtung in die Fermenterflüssigkeit (12) eindrückbar ist.

17. Rühreinrichtung nach Anspruch 16, **dadurch gekennzeichnet, dass** am Beschickungs-Gehäuseendbereich ein Einwurftrichter (26) vorgesehen ist, in dessen Bereich die Schnecke (9) geführt ist.

18. Rühreinrichtung nach Anspruch 16 oder Anspruch 17, **dadurch gekennzeichnet,**
**dass** die erste, obere Gehäuseöffnung (28) in einem Seitenwandbereich des einen Rohrquerschnitt aufweisenden und im Fermenterbehälterinnenraum (2) angeordneten Schneckengehäusebereichs ausgebildet ist, und
**dass** die zweite untere Gehäuseöffnung (11) des Schneckengehäuses (7) in einem Seitenwandbereich oder durch die untere Rohröffnung ausgebildet ist.

19. Rühreinrichtung nach Anspruch 18, **dadurch gekennzeichnet, dass** wenigstens die im Seitenwandbereich ausgebildeten Gehäuseöffnungen (28) mit einem Verschlusselement (30), vorzugsweise einem Schieber, vorzugsweise dicht verschließbar sind.

20. Rühreinrichtung nach einem der Ansprüche 1 bis 19, **dadurch gekennzeichnet,**
**dass** der Fermenterbehälter (3) mittels einem Überlaufrohr (31) mit einem benachbarten Fermenterbehälter (24) strömungsverbunden ist,
**dass** das Überlaufrohr (31) mit einem ersten Überlaufrohrende (32) in einer mittels einem Ausnehmungs-Verschlusselement (33), vorzugsweise einem Schieber, verschließbaren Gehäuseausnehmung (34) des Schneckengehäuses (7) mündet und mit einem zweiten Überlaufrohrende (35) durch eine Fermenterbehälterwand (36) des zweiten Fermenterbehälters (24) in dessen Fermenterbehälterinnenraum (37) geführt ist dergestalt,
**dass** bei geöffnetem Ausnehmungs-Verschlusselement (33) mittels der Schnecke (9) mit Biomasse-Feststoffen versetzte Fermenterflüssigkeit aus dem ersten Fermenterbehälter (3) über das Überlaufrohr (31) in den zweiten Fermenterbehälter (24) pumpbar ist.

21. Rühreinrichtung nach Anspruch 20, **dadurch gekennzeichnet, dass** die im Seitenwandbereich ausgebildeten Gehäuseöffnungen (28) im Pumpbetrieb mittels den diesen zugeordneten Gehäuseöffnungs-Verschlusselementen (30) verschlossen sind und ein Einströmen der Fermenterflüssigkeit (12) in das Schneckengehäuse (7) über eine untere Rohröffnung (11) des Schneckengehäuses (7) erfolgt.

22. Rühreinrichtung nach Anspruch 20 oder Anspruch 21, **dadurch gekennzeichnet, dass** die Gehäuseausnehmung (34) im Nicht-Pumpbetrieb dicht mit dem Ausnehmungs-Verschlusselement (33) verschlossen ist.

23. Rühreinrichtung nach einem der Ansprüche 1 bis 22, **dadurch gekennzeichnet, dass** eine Steuer- und Regeleinrichtung vorgesehen ist, mittels der der Schneckenantrieb (8) und/oder die einzelnen Verschlusselemente (30, 33)entsprechend dem gewünschten, anwählbaren Betriebszustand ansteuerbar sind.
